# EUROPÄISCHE PATENTANMELDUNG

(11) **EP 1 281 969 A2**
(43) Veröffentlichungstag der Anmeldung: **05.02.2003**
(21) Anmeldenummer: 02017491.8
(22) Anmeldetag: 05.08.2002
(51) Int. Cl.: G01N 33/68, G01N 33/543, G01N 21/64, G01N 21/84

(54) **Verfahren und Vorrichtung zum Bestimmen von Proteinen auf einem Reaktionsträger**

(30) Priorität: 03.08.2001 DE 10138072
(71) Anmelder: FRAUNHOFER-GESELLSCHAFT ZUR FÖRDERUNG DER ANGEWANDTEN FORSCHUNG E.V., 80636 München (DE)
(72) Erfinder: Brandenburg, Albrecht, Dr., 79232 March (DE)
(74) Vertreter: Grünecker, Kinkeldey, Stockmair & Schwanhäusser Anwaltssozietät

(57) **Zusammenfassung**

Die vorliegende Erfindung betrifft ein Verfahren zum Nachweisen der Anwesenheit bzw. Abwesenheit eines Proteins/Polypeptids auf einem Reaktionsträger sowie eine Vorrichtung hierfür. Dabei sind insbesondere Reaktionsträger betroffen, die eine Vielzahl von Messpunkten enthalten wie Biochips. Der Nachweis der Proteine erfolgt anhand Ihrer Eigenfluoreszenz.

## Beschreibung

Die vorliegende Erfindung betrifft ein Verfahren zum Nachweisen der Anwesenheit bzw. Abwesenheit eines Proteins/Polypeptids auf einem Reaktionsträger sowie eine Vorrichtung hierfür.

In den letzten Jahren haben sogenannte Hochdurchsatztechnologien zunehmende Bedeutung sowohl in der Analytik wie auch in der Diagnostik gewonnen. Bei diesen Technologien kommen häufig sogenannte Arrays Chips zum Einsatz, bei denen auf einer Fläche von z. B. einem Quadratzentimeter mehrere tausend Molekülspezies aufgebracht sind. Solche Chips wurden ursprünglich zur Analyse von Nukleinsäure entwickelt. Dabei werden Nukleinsäureeinzelstränge, in der Regel DNA, in einzelnen Messfeldern von typischerweise 50 - 100 µm Durchmesser auf den Chip aufgebracht. Jeder dieser Messpunkte repräsentiert eine Molekülspezies, d. h. enthält eine Nukleinsäure mit bestimmter Basensequenz. Jeder dieser Messpunkte prüft daher die ganzflächig auf alle Messpunkte aufgebrachte Probe auf einen Inhaltsstoff hin, auf z. B. die komplementäre Basensequenz. Zur Auslesung der Chips wird in der Regel mindestens einer der Nukleinsäurestränge mit einem Marker, beispielsweise während der PCR, markiert. Als Marker kommen üblicherweise Fluoreszenzfarbstoffe in Betracht, die im sichtbaren Spektralbereich angeregt werden. Häufig für diesen Zweck verwendete Markierungen sind die Cyaninfarbstoffe CY5 und CY3, die im roten (640 nm) bzw. im grünen (530 nm) Spektralbereich angeregt werden. Daneben wird aber auch Fluoreszeinisothiocyanat (FITC) verwendet, das bei 490 nm angeregt wird. Die meisten heute kommerziell erhältlichen Chipreader scannen die Chip-Oberfläche mit einem focusierten Laserstrahl ab und nehmen das Fluoreszenzlicht zeitaufgelöst mit einem Fotomultiplier auf. Vorzugsweise werden rote Halbleiterlaser oder Helium-Neon-Laser, daneben aber auch Argon-Ionen-Laser verwendet. Hersteller solcher Typen sind z. B. die Firmen Agilent, GIS Lumonics und Axon (sämtliche USA). Eine alternative Vorgehensweise besteht in der SPR-Technik (SPR: Surface-Plasmon-Resonance); hierin müssen die Proben keine Markierung tragen. Hier werden die Sonden auf eine dünne Goldschicht aufgebracht, die sich wiederum auf einem Glasträger befindet. Von der Rückseite wird durch Einstrahlen von Licht sogenanntes Oberflächenplasmon in der Goldschicht angeregt.

Die Resonanzfrequenz des Plasmons hängt von der Massenbelegung der Oberfläche des Goldfilms ab. Auf diese Weise ist eine Zunahme der Massenbelegung auf Grund der Anbindung von Molekülen aus einer Lösung nachweisbar. Ein solches Nachweissystem wird von der Firma Biacore (Schweden) hergestellt. Diese Technik kann im Prinzip parallelisiert werden, indem eine größere Oberfläche ausgeleuchtet wird. Das reflektierte Licht kann bildhaft ausgewertet werden, so dass die Massenbelegung ortsaufgelöst erfasst wird. Die Empfindlichkeit dieses Verfahrens ist jedoch deutlich geringer als die des Fluoreszenz-optischen Verfahrens. Außerdem ist der Messfehler, bedingt durch unspezifische Adsorption an der Oberfläche, größer. Nicht zuletzt ist zu bedenken, dass die SPR-Technik nur während oder direkt nach dem Ablauf der Reaktion in Gegenwart der Probe ausgelesen werden kann. Eine Trennung von Nachweisreaktion und Detektion ist kaum möglich.

Die oben genannte DNA-Chiptechnologie kommt insbesondere auch bei der Entschlüsselung der vollständigen Erbinformation ganzer Organismen zum Einsatz.

Nach der zwischenzeitlich abgeschlossenen Entschlüsselung des Humangenoms wird im nächsten Schritt die Frage nach dem Bedeutungsgehalt der erzeugten genomischen Information von Bedeutung. Hierzu werden zur Zeit in großem Umfang sogenannte Expressionsanalysen durchgeführt, bei denen die aus der genomischen DNA erzeugten RNA-Sequenzen eines bestimmten Gewebezelltyps etc., analysiert werden. Erst durch die von RNAs kodierten Proteine wird jedoch eine biologische Wirkung in der Zelle bzw. im Organismus entfaltet. Demzufolge wird es in Zukunft zunehmend wichtiger, dass möglichst vollständige Proteinmuster einer Zelle zu analysieren. Ferner werden wichtige Vorgänge in der Zelle durch Bindung von Proteinen an Nukleinsäuren gesteuert. Häufig ist es auch ein Muster an Proteinen, das an bestimmte Nukleinsäuren binden muss, um eine bestimmte biologische Wirkung hervorzurufen. Demzufolge werden die Hochdurchsatztechnologien zunehmend wichtiger für die Analyse der Proteinmuster eines gegebenen Systems.

Bei der DNA-Chiptechnologie wird im wesentlichen ein fremdes Markermolekül in die auf dem Chip aufgebrachten Nukleinsäuremoleküle eingeführt. Diese chemische Modifikation der Nukleinsäuremoleküle ist in der Regel relativ einfach und kann beispielsweise im Rahmen der PCR-Technologie gleichzeitig mit der Amplifikation der Nukleinsäure durchgeführt werden. Für die Analyse von Nukleinsäuren hat sich die Fluoreszenzanalyse als empfindliche und handhabbare Nachweistechnik auch im Rahmen der Chiptechnologie etabliert.

Für die Untersuchung von Proteinen auf Chipebene können die Erfahrungen aus der DNA-Chiptechnologie jedoch nur teilweise übernommen werden. Zwei gravierende Probleme sind zur Zeit vorrangig. Erstens existiert keine Amplifikationstechnik für Proteine, mit denen man analog zur PCR-Technik die zur Verfügung stehende Anzahl von Probenmolekülen vergrößern könnte (um somit die Nachweisgrenze eines Moleküls in einer biologischen Probe um mehrere Größenordnungen zu erniedrigen). Zweitens ist für die Anbindung von Markermolekülen, wie z. B. Fluoreszenzfarbstoffen, ein separater Schritt notwendig, durch den sich unter Umständen die native Konformation, und so z. B. die Bindungseigenschaften, der Proteine verändem können.

Da sich jedoch in zunehmendem Maße ein hoher Bedarf an dem Einsatz der Chiptechnologie zur Analyse von Proteinen ergibt, lag der vorliegenden Erfindung das technische Problem zugrunde, ein Verfahren anzugeben, das hochempfindlich ist, möglichst einfach in der Handhabung ist und gleichzeitig die nachzuweisenden Proteine möglichst in der nativen Konformation belässt. Ferner sollte eine möglichst hohe räumliche Auflösung gewährleistet sein, damit das Verfahren auch auf Proteinchips anwendbar ist.

Dieses Problem wird erfindungsgemäß gelöst durch ein Verfahren zum Bestimmen der Anwesenheit bzw. Abwesenheit mindestens eines Proteins/Polypeptids an einem vorgegebenen Messpunkt auf einem Reaktionsträger, wobei der Reaktionsträger einer elektromagnetischen Strahlung (dem Anregungslicht) ausgesetzt wird, die die Eigenfluoreszenz eines Proteins anregen kann.

Bei dem Verfahren kann grundsätzlich die Anwesenheit bzw. Abwesenheit eines Polypeptids in einer biologischen Probe ermittelt werden. Dabei wird die zu untersuchende biologische Probe auf einen Träger aufgebracht, der einen Bindungspartner in einem vorgegebenen Messpunkt für ein gewünschtes Polypeptid enthält. Liegt nun das fragliche Polypeptid in der Probe vor, kann es nach Aufbringen der Probe an seinen Bindungspartner binden. Das so an dem vorgegebenen Messpunkt durch seinen Bindungspartner fixierte Protein lässt sich dann nach dem Bestrahlen mit Anregungslichts anhand seiner Eigenfluoreszenz nachweisen. Als Bindungspartner für das Polypeptid an einem vorgegebenen Messpunkt kommen letztendlich beliebige Substanzen in Betracht, sofern sie denn eine Wechselwirkung mit dem nachzuweisenden Polypeptid eingehen können. Hierfür kommen beispielsweise niedermolekulare organische Substanzen in Betracht aber auch Nukleinsäuren sowie andere Polypeptide. Vorzugsweise sollten diese Bindungspartner jedoch eine möglichst geringe Eingenfluoreszenz nach Bestrahlung mit dem Anregungslicht aufweisen. Insbesondere sollte der Bindungspartner möglichst keinen Tryptophan-, Tyrosinoder Phenylallaninrest enthalten. Der Reaktionsträger enthält mindestens einen solchen Messpunkt mit einem Bindungspartner für ein Polypeptid, vorzugsweise enthält der Reaktionsträger jedoch mehrere Messpunkte bis hin zu mehreren tausend Messpunkten pro Quadratzentimeter, wobei jeder Maßpunkt vorzugsweise mit einem anderen Bindungspartner belegt ist, so dass möglichst verschiedene Polypeptide an die verschiedenen Messpunkte binden können.

Wie für den Bindungspartner erläutert, sollte auch der Reaktionsträger eine möglichst geringe Eigenfluoreszenz nach Bestrahlen mit dem Anregungslicht abgeben. In Frage kommen z. B. Quarzglas, Keramik, Silizium aber auch beschichtete Materialien wie Gold-beschichtetes Glas. Bei dem erfindungsgemäßen Verfahren wird die Eigenfluoreszenz des Polypeptids ausgenutzt ohne dass eine weitere Messgröße abgenommen werden müsste.

Bei einer bevorzugten Ausführungsform ist der Reaktionsträger ein sogenannter "Biochip". Hierunter werden Träger verstanden, die eine hohe Dichte an Messpunkten, jeder Messpunkt enthaltend eine bestimmte Molekülspezies (Liganden/Bindungspartner), aufweisen. Solche Chips werden häufig auch als Arrays bezeichnet. Sofern der Chip der Analyse von Nukleinsäuremolekülen dient, spricht man auch von DNA-Chips. Wird der Chip vorzugsweise zur Analyse von Proteinen verwendet, spricht man auch von Proteinchips. Die Analyse von Proteinmustern mittels der Chiptechnologie stößt neben den beschriebenen Schwierigkeiten des Einführens von Markierungen in die Moleküle auch auf größere Probleme bei der eigentlichen Analyse der Probe. Wie oben ausgeführt, sollte es zu einer möglichst spezifischen Wechselwirkung zwischen dem bzw. den Polypeptiden einer Probe und dem zugehörigen Bindungspartner im Messpunkt des Chips kommen. Zu diesem Zweck sollten die Polypeptide möglichst in nativem Zustand bleiben, so dass deren Bindungseigenschaften möglichst unbeeinträchtigt von der Probenhandhabung bleiben. Ansonsten kann es leicht zu falschen Ergebnissen kommen, da es durch Konformationsänderungen der Polypeptide zu künstlichen Bindungsreaktionen kommen kann bzw. eine an sich vorhandene Bindungsaktivität zerstört wird und das dann bestimmte Fluoreszenzsignal zu Fehlinterpretationen hinsichtlich der Proteinzusammensetzung führt. Gleiches gilt auch für die Bindungspartner, die in den Messpunkten auf dem Chip vorliegen. Verfahren zum schonendem Aufbringen von Proteinen auf Biochips sind jedoch bereits aus dem Stand der Technik bekannt, wie beispielsweise beschrieben in der US 5,741,700, US 6,079,283 bzw. US 6,083,762. Eine Übersicht über die neuesten Entwicklungen auf dem Gebiet der Proteinchiptechnologie befindet sich auch in Pharmaco Genomics 2000, November, 1(4), Seiten 395 - 416 von Weinberger S. R., et al.

Solche Proteinchips finden z. B. Anwendung beim Auffinden gewünschter Wirkstoffe in chemischen Substanzbibliotheken. Vorzugsweise wird beispielsweise eine chemische Substanzbibliothek auf einen Reaktionsträger aufgebracht und dieser dann anschließend mit Polypeptid-haltigen Proben (z. B. einem gewünschten Target für einen pharmazeutischen Wirkstoff) in Kontakt gebracht. Kommt es dann zur Bindung eines Polypeptids (Drug-Targets) an ein Mitglied der Substanzbibliothek, erzeugt der entsprechende Polypeptid-haltige Messpunkt nach Bestrahlung mit dem Anregungslicht ein Fluoreszenzmesssignal. Analog kann der Chip auch ein Muster an verschiedenen Nukleinsäuren enthalten, die darauf untersucht werden, ob sie an vorgegebene Polypeptide binden können. Kommt es zu einer spezifischen Wechselwirkung eines Polypeptids mit dem dazugehörigen Polypeptid, erzeugt ein solcher Messpunkt ebenfalls ein Messsignal. Schließlich kann ein Chip jedoch auch in jedem Messpunkt identische Bindungspartner für ein Polypeptid enthalten und auf jeden Messpunkt wird eine andere Polypeptidspezies gegeben. Auf diese Weise lassen sich in einem Pool zahlreicher verschiedener Polypeptide jene identifizieren, die mit einem vorgelegten Bindungspartner binden. Auf diese Weise lassen sich beispielsweise Proteine identifizieren, die an bestimmte Promotorsequenzen binden. Ferner lassen sich mit dieser Technologie auch beliebige Rezeptor-Liganden-Wechselwirkungen testen. Schließlich lassen sich auch Protein-Protein-Wechselwirkungen in effizienter Weise austesten, indem der Array z. B. verschiedene Bindungsepitope für ein gegebenes Protein enthält. Als Alternative kann der Reaktionsträger auch einen Träger mit mehreren Vertiefungen darstellen, in denen die Reaktionen zwischen Probe und Sonde ablaufen. Zu solchen Trägern zählt z. B. eine bekannte Mikrotiterplatte oder "miniaturisierte" Wellplatten.

Ein wesentlicher Vorteil des erfindungsgemäßen Verfahrens besteht darin, dass das zu bestimmende Polypeptid keine Fremdmarkierung, wie einen zusätzlichen Fluoreszenzmarker, enthalten muss. Das Einführen solcher Marker in Proteinmoleküle ist aufwendig und beinhaltet stets das Risiko, dass das markierte Protein zumindest teilweise denaturiert wird, so dass sich die Bindungseigenschaften des Proteins ändern können.

Bei einer weiteren bevorzugten Ausführungsform wird die Eigenfluoreszenz des Polypeptids dadurch gemessen, dass die Fluoreszenz von darin enthaltenen Tryptophan-, Tyrosin- bzw. Phenylalaninresten nach Anregung mit dem Anregungslicht gemessen wird. Insbesondere ist es bevorzugt, die Eigenfluoreszenz von Tryptophan auszunutzen. Die drei genannten aromatischen Aminosäuren leisten einen verschiedenen Beitrag zur Eigenfluoreszenz der Proteine. Die Absorptionsmaxima liegen für Tryptophan bei 278 nm, Tyrosin 274,25 nm und Phenylalanin 257,5 nm. Die Extinktionen verhalten sich wie 27 : 7 : 1. Die Fluoreszenzemissionsspektren liegen im Wellenlängenbereich von 300 bis 350 nm und werden im wesentlichen von Tryptophan bestimmt. Demgegenüber haben Adenin, Cytosin, Guanin und Thymin Absorptionsbanden bei 260 nm. Die Fluoreszenz dieser Substanzen liegt jedoch um 3 Größenordnungen niedriger.

Bei einer weiter bevorzugten Ausführungsform wird als Anregungslicht ein Licht mit einer Wellenlänge von 270 bis 290 nm, vorzugsweise ca. 280 nm verwendet. Die Anregung bei 280 nm bedingt allerdings eine Optik mit entsprechender Transmission für diesen Wellenlängenbereich. Hierfür kommt beispielsweise Quarzglas in Frage, das bis zu ca. 180 nm eine Transmission von über 90 % zeigt.

Bei einer weiteren bevorzugten Ausführungsform wird die Eigenfluoreszenz des Polypeptids in den jeweiligen Messpunkten mittels ortsaufgelöster Fluoreszenzmessung bestimmt.

Bei einer weiteren bevorzugten Ausführungsform wird das Anregungslicht von einem Laser erzeugt.

Bei einer weiteren bevorzugten Ausführungsform umfasst der Reaktionsträger einen planaren Lichtwellenleiter, in den das Anregungslicht eingekoppelt werden kann. Herkömmliche Träger wie sie bei der DNA-Chiptechnologie eingesetzt werden (als Materialien kommen beispielsweise Glas- oder Kunststoffe wie PMMA zum Einsatz), sind für das erfindungsgemäße Verfahren weniger geeignet, da sie eine relativ starke Eigenfluoreszenz bei der eingesetzten Anregungswellenlänge besitzen. Bevorzugte Materialien sind daher Quarzglas, Silizium sowie Keramik oder ausgewählter Kunststoffe (im wesentlichen Zusatzstoff-freie Kunststoffe, aber auch Gläser oder Polymere, die z. B. eine Beschichtung der Art tragen, dass das Anregungslicht nicht in den Träger eindringt und so eine Eigenfluoreszenz im wesentlichen unterbunden wird (z. B. geschwärzte Gläser)). Auch lassen sich durch Veredelungstechniken, wie die Beschichtung billiger, aber von ihrer Eigenfluoreszenz her eigentlich nicht geeigneter Materialien wie Glas, mit einem günstigen Kostenfaktor geeignete Träger herstellen. Die Beschichtungsmaterialien sollten allerdings biokompatibel sein, insbesondere sollten Sie keine negativen Auswirkungen auf die native Konformation der aufgebrachten Proteine ausüben. Als Beschichtungsmaterialien kommt beispielsweise Gold in Betracht.

Bei dem erfindungsgemäßen Verfahren kann das Anregungslicht letztlich auf beliebige Art und Weise dem Reaktionsträger, und somit dem darauf befindlichen Polypeptid, zugeführt werden. Vorzugsweise wird das Anregungslicht dem Reaktionsträger mittels einer Optik zugeführt, so dass das Licht auf die gewünschte Position auf den bzw. im Reaktionsträger gelenkt wird. Alternativ kann das Anregungslicht dem Reaktionsträger auch direkt flächenhaft zugeführt werden, wobei ein mehr oder weniger großes Areal des Trägers unmittelbar angestrahlt wird. Weiterhin kann das Anregungslicht auch in einem planaren Lichtwellenleiter, der Bestandteil des Chips ist, eingekoppelt werden, so dass es gleichzeitig allen Messpunkten zugeführt wird.

Hinsichtlich der Abnahme des Fluoreszenzlichts stehen schließlich auch zahlreiche Möglichkeiten zur Verfügung. Bei einer bevorzugten Variante wird das vom Polypeptid emmitierte Fluoreszenzlicht mittels einer Optik einem Detektor zugeführt; alternativ kann das emmitierte Fluoreszenzlicht direkt einer Kamera zugeführt werden. Ferner kann das emmitierte Fluoreszenzlicht durch zeilenweises Scannen des Reaktionsträgers abgenommen und einem Detektor zugeführt werden. Schließlich kann das Fluoreszenzlicht auch mittels einer Abbildungsoptik einem Detektor zugeführt werden. Die oben angeführten Alternativen zum Zuführen des Anregungslichts einerseits und zum Abnehmen des Fluoreszenzlichts andererseits lassen sich jedoch beliebig kombinieren, wobei zumindest eine Ortsauflösung auf Anregungsseite oder Detektionsseite erfolgen muss.

Bei einer weiteren bevorzugten Ausführungsform ist die Optik, mit der das Anregungslicht dem Reaktionsträger zugeführt wird, für ein Licht der Wellenlänge von ca. 270 bis 290 nm, vorzugsweise für ein Licht der Wellenlänge von ca. 280 nm durchlässig, wobei die Transmission des Materials bei diesen Wellenlängen zumindest bei 90 % liegen sollte.

Bei einer weiteren bevorzugten Ausführungsform wird Fluoreszenzlicht mit einer Wellenlänge im Bereich von 300 bis 450 nm detektiert.

Die erfindungsgemäße Vorrichtung zum Durchführen des oben beschriebenen Verfahrens enthält eine Optik, mit der das Anregungslicht dem Reaktionsträger zugeführt wird, wobei die Optik durchlässig ist für ein Anregungslicht mit einer Wellenlänge im Bereich von 270 bis 290 nm, vorzugsweise für ein Anregungslicht mit der Wellenlänge von ca. 280 nm. Bei einer bevorzugten Ausführungsform handelt es sich bei der Optik um eine Quarzoptik.

Die folgenden Beispiele und Zeichnungen erläutem die Erfindung:
Figur 1a zeigt einen Biochip in schematisch perspektivischer Darstellung;
Figur 1b zeigt einen Ausschnitt eines Biochip nach Figur 1 für einen Messpunkt einer Oberfläche mit immobilisierten DNA-Einzelsträngen;
Figur 2 zeigt eine Darstellung der Trennung von gebundener und flüssiger Phase nach dem ATR-Prinzip;
Figur 3 zeigt eine schematische Darstellung einer Einrichtung zum Auslesen eines ATR-Prismas durch Einfachreflexion;
Figur 4 zeigt eine schematische Darstellung zum Auswerten eines ATR-Prismas durch 4-fach-Reflextion;
Figur 5 zeigt eine schematische Darstellung der Einrichtung unter Verwendung des Prinzips einer "homogenisierten" Vielfachreflexion (Flächenausleuchtung);
Figur 6 zeigt eine schematische Darstellung der Einrichtung unter Verwendung eines planaren Lichtwellenleiters;
Figur 7 zeigt eine graphische Darstellung zum zeilenweisen Scannen eines Biochips;
Figur 8 zeigt eine graphische Darstellung der flächenhaften Ausleuchtung eines Biochips mit einem Bildaufnahmesystem;
Figur 9 zeigt eine schematische Darstellung zum zeilenweisen Auslesen des Biochips

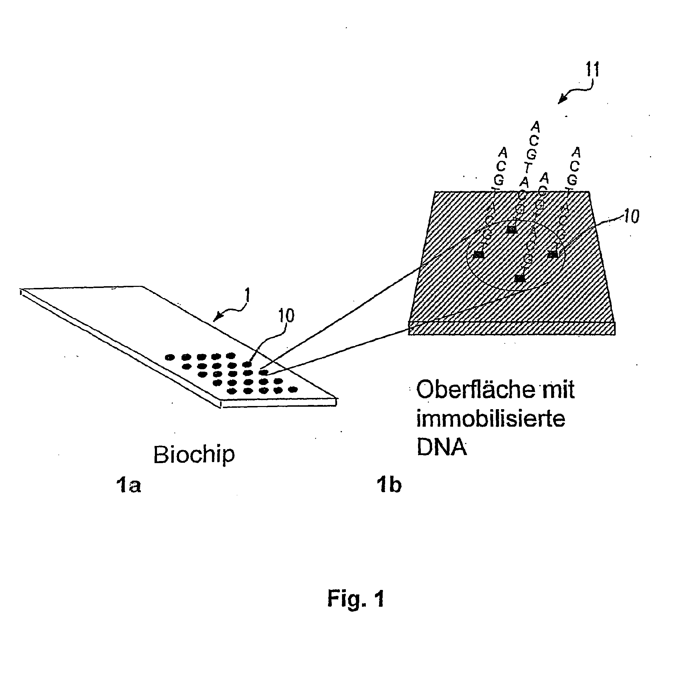

In Fig. 1a ist in schematischer Darstellung ein solcher Biochip 1 gezeigt, der ein kleines Plättchen bildet, auf dessen Oberfläche eine Vielzahl von Nukleinsäuren 11 in einzelnen Messpunkten 10 immobilisiert sind. An jedem einzelnen Messpunkt 10 befindet sich ein Oligonukleotid mit einer definierten Basensequenz. Dies ist in Fig. 1b dargestellt. Bei dem erfindungsgemäßen Verfahren kann die angegebene Nukleinsäure auch ersetzt sein durch einen anderen Bindungspartner (z. B. ein weiteres Protein) für ein zu bestimmendes Protein. Nach Zugabe der proteinhaltigen Lösung bindet ein Protein spezifisch an den immobilisierten Bindungspartner die immobilisierte DNA. An diesem Messpunkt lässt sich dann eine Eigenfluoreszenz des Polypeptids detektieren.

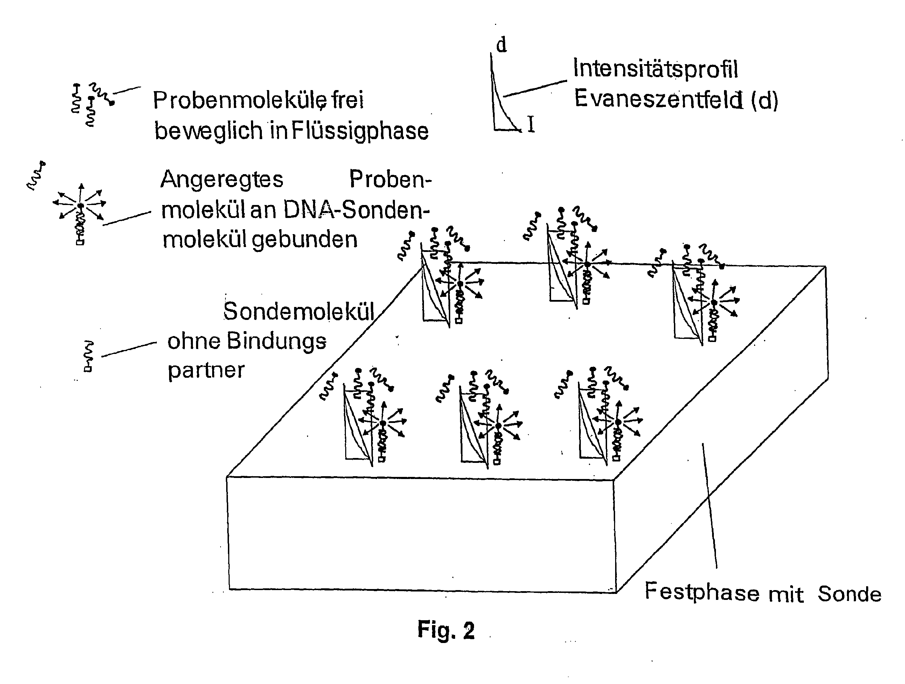

Die folgenden Ausführungsbeispiele verwenden die Totale Interne Reflexions Fluoreszenz (TIRF). Durch die Totalreflexion eines Lichtstrahles an der Grenzfläche zwischen zwei optisch unterschiedlich dichten Medien wird im optisch dünneren Medium ein elektromagnetisches Feld erzeugt. Das optisch dichtere Medium sei hier eine Festphase und das optisch dünnere Medium eine Flüssigphase. Dieses sogenannte Evaneszentfeld dringt nur einige hundert nm von der Grenzfläche in das flüssige Umgebungsmedium ein. Dadurch werden fast ausschließlich die an der Oberfläche angebundenen Fluoreszenzfarbstoffe (Polypeptide) nachgewiesen. Die im Umgebungsmedium gelösten Polypeptide tragen nur geringfügig zum Messsignal bei, wie es in Fig. 2 dargestellt ist. Das ermöglicht die Messung des zeitlichen Verlaufs von Reaktionen.

In Fig. 2 ist deutlich dargestellt, dass die Intensitätsprofile der Evaneszentfelder sehr stark abfallen.

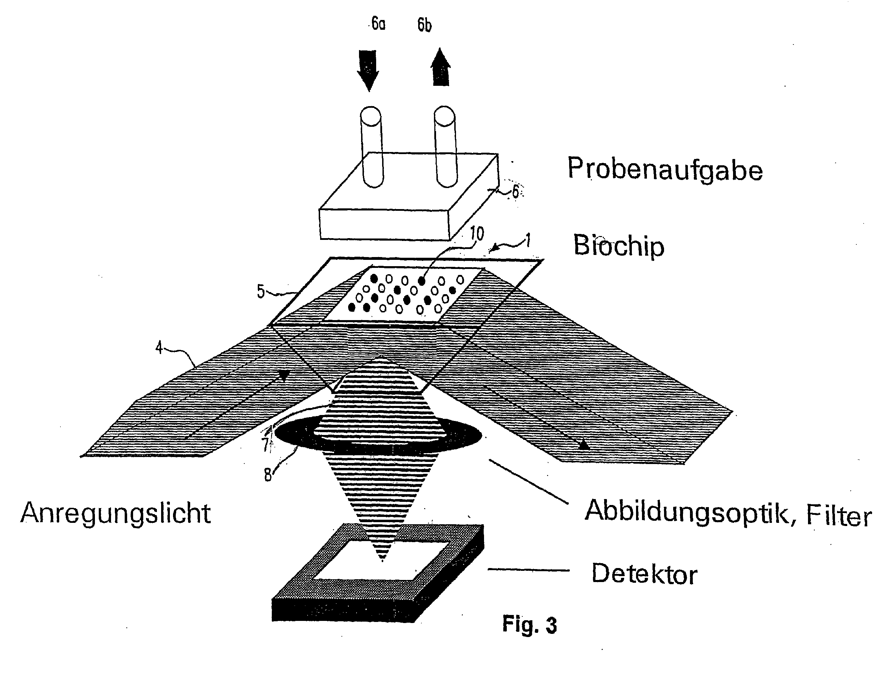

Bei der Anordnung nach Fig. 3 wird das Messfeld auf der Oberseite des Prismas flächig ausgeleuchtet.

Dabei bringt eine Durchflusszelle die Flüssigphase mit der Festphase in Kontakt. Eine Flusszelle 6 ist gekoppelt mit einem Fluidik-System zur Handhabung der Flüssigphase. Durch den permanenten Kontakt des Bindungspartners, also z. B. der an die Festphase gebundenen Nukleinsäure, mit der flüssigen Phase, ist die Regenerierbarkeit des Biochips gegeben. Als Messchip wird ein transparentes Prisma 5 verwendet.

Fig. 3 zeigt des weiteren in schematischer Darstellung eine Einrichtung zum Auslesen des Biochips 1, der eine Konfiguration aufweist, wie er bereits oben beschrieben worden ist. Der Biochip 1 besteht wiederum aus einem transparenten Substrat und vorzugsweise einer auf das Substrat aufgebrachten hochbrechenden Beschichtung als planarer Lichtwellenleiter. Der Lichtwellenleiter trägt ein Feld von Messpunkten 10, und ein Anregungslicht 4 wird über das Prisma 5 in den Lichtwellenleiter eingekoppelt und in diesem geführt. Die Messsonde ist so ausgebildet, wie dies anhand der Fig. 1b erläutert wurde.

Zur Analyse von den Polypeptiden in einer Probe wird diese Probe hier in der Flusszelle 6 und durch diese hindurchgeführt, wie dies durch die Strömungspfeile 6a und 6b angedeutet ist. Die Flusszelle 6 wird abgedichtet auf den Lichtwellenleiter aufgesetzt und umgibt das Messfeld mit den Messpunkten 10 rahmenartig und fluiddicht, so dass die Probe mit allen Messpunkten 10 zur möglichen Bindung in Wechselwirkung treten kann. Die Detektion der durch das Evaneszentfeld des Anregungslichtes 4 angeregten Fluoreszenzstrahlung 7 erfolgt z.B. mittels einer Abbildungsoptik 8 in Verbindung mit einem hier nicht gezeigten Filter und einem ortsauflösenden Detektor 9, wie z.B. einer CCD-Kamera oder einem Foto-Multiplier.

Auf diese Weise ist eine Erfassung der Polypeptidbindung an den Chip und eine parallele Auslesung des Biochips 1 mit allen Messpunkten 10 gleichzeitig möglich. Zugleich erfolgt eine selektive Anregung der gebundenen Polypeptide in den Messpunkten 10. Durch dieses Messprinzip ist daher ohne besondere Probenvorbereitung eine mit großer Genauigkeit hinsichtlich der Ortsauflösung und auch hinsichtlich der Präsenz von gebundenen Polypeptiden eine sehr schnelle Auswertung und Detektion des Biochips 1 möglich.

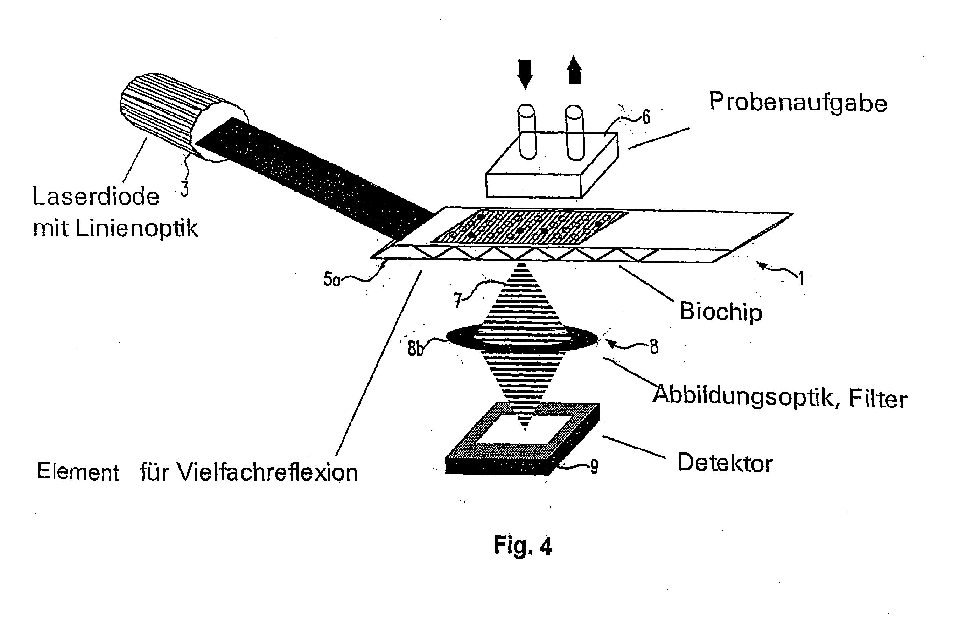

Bei der Anordnung gemäß Fig. 4 wird eine Kante eines wesentlich dünneren Prismas 5a, mit einer Stärke von ungefähr 1 mm mit einer Linienoptik ausgeleuchtet. Dabei sind mehrere Chips 5a nebeneinander angeordnet. Durch Vielfachreflexion an den Ober- und Unterseiten des Chips 5a wird eine flächige Ausleuchtung des Messfeldes erzeugt. Die Detektion der emittierten Fluoreszenzstrahlung erfolgt mit einem ortsauflösenden Detektor 9. In diesem Fall dient als Lichtquelle eine Laserdiode 3 oder gefiltertes Weißlicht (breitbandige Lichtquellen z. B. Halogen- oder Gasentladungslampen) oder eine andere Laserquelle (z. B. Gas- oder Festkörperlaser).

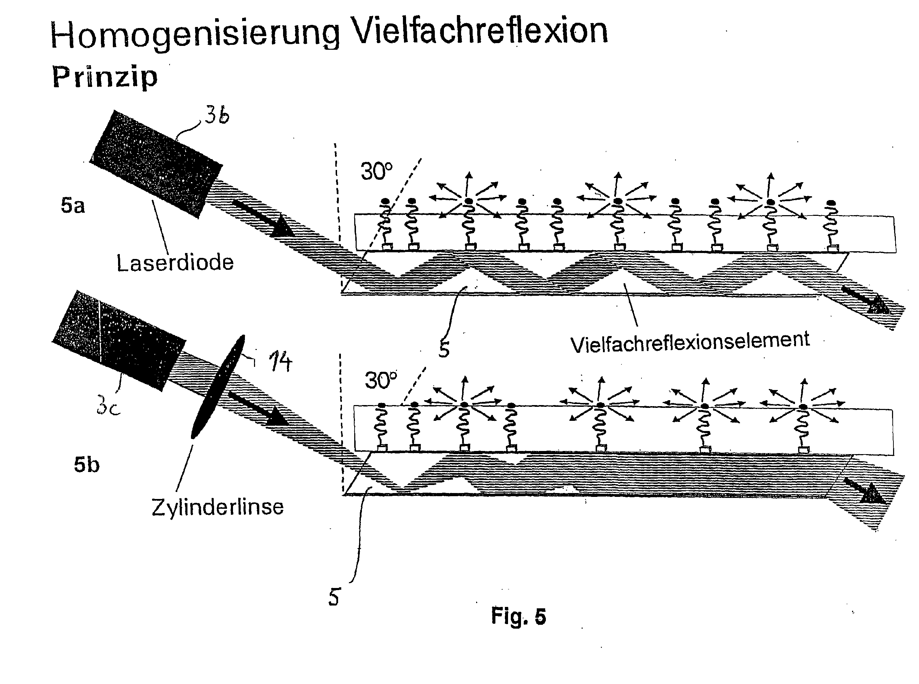

Fig. 5 zeigt in den Fig. 5a und 5b jeweils die Einstrahlungsverhältnisse in ein Vielfachreflexionselement 5 (wie z. B. einen Chip) als Reaktionsträger unter Darstellung des Laserstrahles mit TIRF (Fig. 5a) unter Einstrahlung durch eine Laserdiode 3a und in Fig. 5b die Möglichkeit, tatsächlich zu einer flächigen Ausleuchtung der Oberfläche des Elements 5 dadurch zu gelangen, dass die Einstrahlung des Lichtes durch die Lichtquelle 3c über eine Zylinderlinse 14 in das Element 5 erfolgt.

Wie dargestellt, wird hierdurch eine tatsächlich im wesentlichen vollflächige Ausleuchtung der Prismenoberfläche erreicht, während bei der Ausleuchtung des Randes des Elements 5 mit einem kollimierten Laserstrahl dieses dazu führt, dass die Ober- und Unterseite des Elements 5 nur an bestimmten Stellen selektiv ausgeleuchtet wird und Messpunkte, die in nicht ausgeleuchteten Bereichen liegen, nicht ausgewertet werden können. Demgegenüber wird bei der Fokussierung des Lichtstrahles mit einer Zylinderlinse 14 auf die Kante des Elements 5 erreicht, dass der Lichtstrahl im Element 5 divergent weiterläuft. Nach einer gewissen Wegstrecke ist der Strahl so weit aufgeweitet, dass praktisch eine homogene Ausleuchtung der Biochip-Oberfläche gegeben ist.

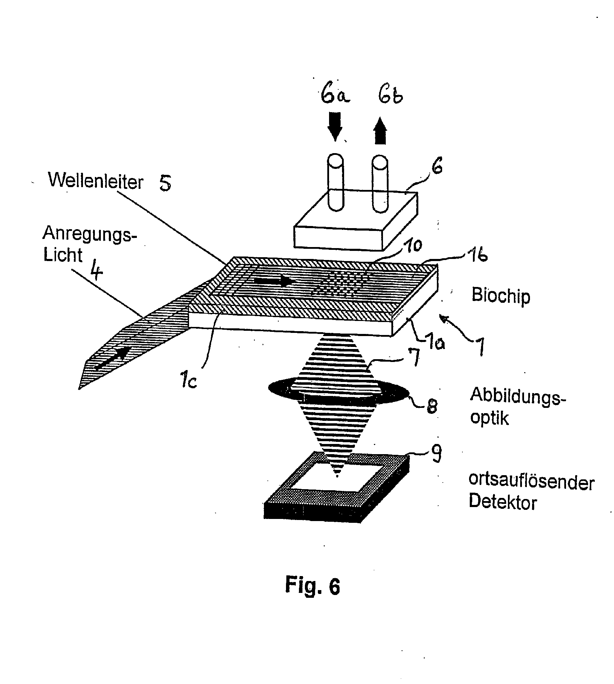

Fig. 6 zeigt in schematischer Darstellung eine Einrichtung zum Auslesen eines Biochips 1, der eine Konfiguration aufweist, wie sie in Fig. 2 dargestellt ist. Der Biochip 1 besteht wiederum aus dem transparenten Substrat 1a und der auf das Substrat aufgebrachten hochbrechenden Beschichtung als planarer Lichtwellenleiter 1b. Der Lichtwellenleiter trägt ein Feld von Messpunkten 10 und das Anregungslicht 4 wird über ein Koppelgitter 5 in den Lichtwellenleiter 1b eingekoppelt und in diesem geführt. Die Messpunkte 10 sind so ausgebildet, wie dies anhand der Fig. 1b und Fig. 2 erläutert wurde. Zur Analyse von Polypeptiden in einer Probe wird diese Probe hier beispielsweise in einer Flusszelle 6 und durch diese hindurchgeführt, wie dies durch die Strömungspfeile 6a, 6b angedeutet ist. Die Flusszelle 6 wird abgedichtet auf den Lichtwellenleiter 1b aufgesetzt und umgibt das Messfeld mit den Messpunkten 10 rahmenartig und fluiddicht, so dass die Probe mit allen Messpunkten 10 zur möglichen Bindung in Wechselwirkung treten kann. Die Detektion der durch das Evaneszentfeld des Anregungslichtes angeregten Fluoreszenzstrahlung 7 erfolgt z.B. mittels einer Abbildungsoptik 8 in Verbindung mit einem (hier nicht gezeigten) Filter und einem ortsauflösenden Detektor 9, wie z.B. einer CCD-Kamera oder einem Foto-Multiplier. Die Detektion kann aber auch durch Abstrahlung des Fluoreszenzlichtes nach oben oberhalb des Wellenleiters 1b erfolgen.

Auf diese Weise ist eine "in-situ-Erfassung" der Bindung und eine parallele Auslesung des Biochips 1 mit allen Messpunkten 10 gleichzeitig möglich, wie dies im übrigen auch bei den Anordnungen nach Figur 3 - 5 gegeben ist. Zugleich erfolgt eine selektive Anregung der gebundenen Polypeptide in den Messpunkten. Durch dieses Messprinzip ist daher ohne besondere Probenvorbereitung eine mit großer Genauigkeit hinsichtlich der Ortsauflösung und auch hinsichtlich der Präsenz von gebundenen Polypeptiden eine sehr schnelle Auswertung und Detektion des Biochips 1 möglich.

Bekanntermaßen muss bei einer Analysenanordnung nach Fig. 6 zusätzlicher Aufwand für die Einkopplung des Anregungslichtes 4 in den Wellenleiter 1b (hier über ein Gitter 5) getrieben werden. Einerseits werden somit zusätzliche Präparationsschritte bei der Herstellung des Biochips 1, andererseits sind Justiervorrichtungen in der optischen Anordnung zwischen Anregungslichtquelle (Laserquelle) und Biochip erforderlich. Die damit einhergehende Erhöhung der Biochipkosten, die als Verbrauchsmaterial problematisch ist, kann dadurch vermieden werden, dass eine Mess- und Analysenanordnung nach der schematischen Darstellung gemäß Fig. 4 verwendet wird, bei der eine Anregung der Fluoreszenz auf der Oberseite des Lichtwellenleiters 1b z.B. von der Rückseite des Biochips 1 und damit von der gegenüberliegenden Seite in Bezug auf die Flusszelle 6 erfolgt, aber eine Detektion des von den gebundenen Polypeptide emittierten Fluoreszenzlichtes durch Einkopplung desselben in den planaren Wellenleiter 1b vorgesehen wird.

Als alternative Lösung wird das von einer Laserstrahlquelle emittierte Anregungslicht vorzugsweise über eine Ablenkeinheit (nicht gezeigt) auf einen Umlenkspiegel (nicht gezeigt) und von dort in den Wellenleiter 1b im Bereich des Messfeldes der Messpunkte 10 geführt, an dem sich z.B. die Flusszelle 6 befindet. Hierbei findet mit einer Scanneinrichtung eine zweiachsige Relativbewegung zwischen Anregungslichtstrahl und Biochip statt. Auch in diesem Fall wird durch den planaren Wellenleiter 1b eine Trennung von angebundenen und gelösten Fluorochromen erreicht, da nur das im Bereich des Evaneszentfeldes des Anregungslichtes 4 emittierte Fluoreszenzlicht 7 auch in den planaren Wellenleiter 1b eingekoppelt und anschließend detektiert wird. Die gelösten Polypeptide erzeugen keine Untergrundintensität, da dieses Licht nicht an die Erfassungseinrichtung herangeführt wird, sondern nur das in dem planaren Wellenleiter 1b geführte Fluoreszenzlicht der gebundenen Polypeptide. Die Strömungspfeile 6a, 6b geben wiederum den Probenfluss durch die Flusszelle 6 an, während die Abbildungsoptik 8 mit Filter im Anschluss an den planaren Lichtwellenleiter 1b dargestellt ist, wobei als ortsauflösender Detektor hier ein Foto-Multiplier verwendet wird.

Da bei diesem Ausführungsbeispiel eine zeilenweise Auslesung erfolgen muss, wird der Biochip 1 in Pfeilrichtung A, wie angegeben, entsprechend bewegt.

Gegebenenfalls kann auch auf der anderen Seite des Wellenleiters eine Erfassungseinrichtung mit Auswerteoptik, Filter und Foto-Multiplier zur Erfassung des auf der anderen Seite aus dem Lichtwellenleiter 1b austretenden Fluoreszenzlichtes vorgesehen sein oder der Detektor kann direkt an die Kante des Lichtwellenleiters 1b angekoppelt sein.

Als Lichtwellenleiter kann auch unmittelbar eine Quarzglasplatte verwendet werden, die selbst den Reaktionsträger und zugleich planaren Lichtwellenleiter bildet, ähnlich wie in Figur 4. In diesem Fall kann eine separate, den Lichtwellenleiter bildende Beschichtung eines Substrats entfallen.

Die erfindungsgemäße Lösung gestattet die Real-Time-Messung und Auswertung von Biochips oder auch von anderen Reaktionsträgern, auf deren mit einem planaren Wellenleiter beschichteten Oberseite durch Reaktion die Analysen von Substanzen in Proben bewirkt werden.

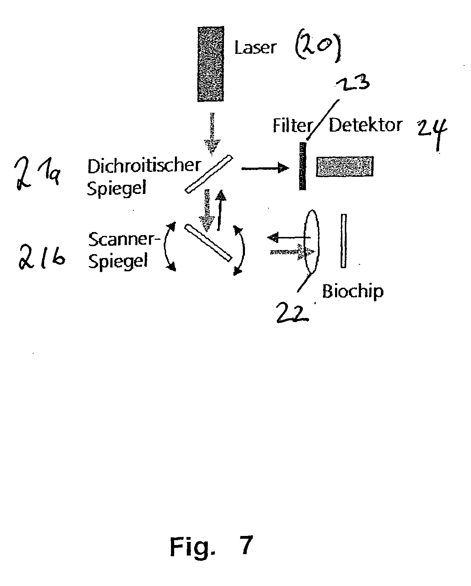

Bei einer weiteren bevorzugten Ausführungsform erfolgt die Anregung mittels Laserlicht, das dem Biochip über ein Spiegelsystem (21a; 21b) sowie einem Linsensystem (22) zugeführt wird. Dabei kann die Oberfläche des Chips durch Verstellen eines Scannerspiegels (21b) abgetastet werden. Das imitierte Fluoreszenzlicht wird dann ebenfalls über ein Linsensystem und Spiegelsystem einem Detektor zugeführt (siehe Figur 7).

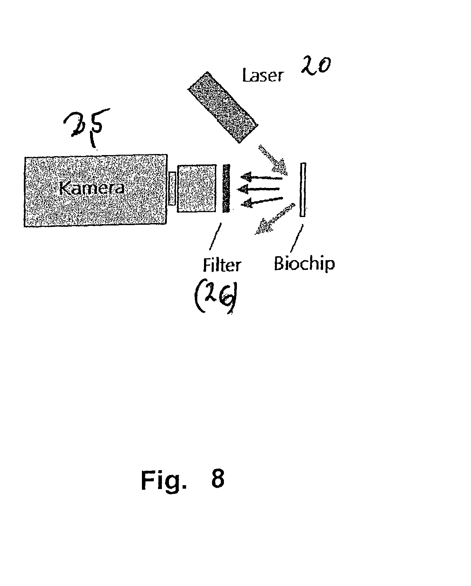

Bei einer weiteren bevorzugten Ausführungsform wird mittels einer Auflichtbeleuchtung durch Laserlicht das Probenmaterial auf dem Biochip angeregt und das Fluoreszenzlicht über einen Filter direkt einer Kamera zugeführt. Bei dieser Variante befindet sich die Ortsauflösung auf der Seite der Detektion (siehe Figur 8).

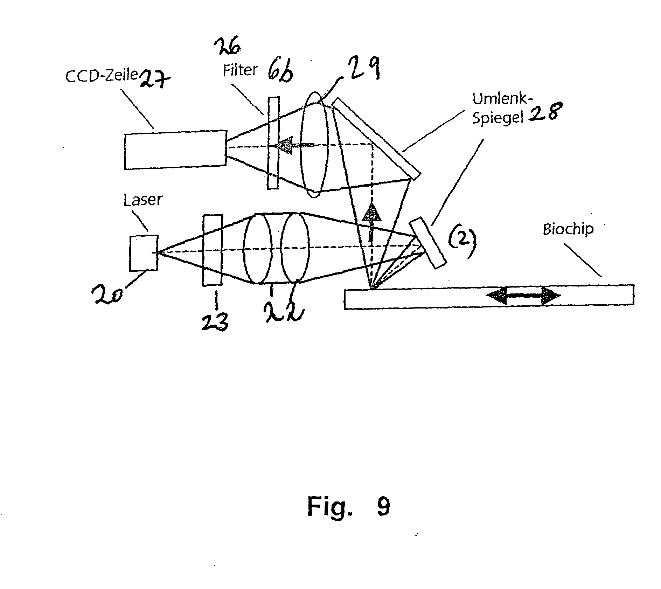

Bei einer weiteren bevorzugten Ausführungsform kommt es zu einer zeilenweisen Anregung bzw. Auslesung, indem der Biochip in der entsprechenden Richtung durch den Anregungsstrahl geführt wird, bzw. das Detektionssystem den Biochip zeilenweise ausliest (siehe Figur 9).

Bei einer weiteren bevorzugten Ausführungsform enthält der Reaktionsträger in jedem Messpunkt gleichartige Sonden, und diesem Reaktionsträger werden verschiedene Proben bzw. verschiedene Proteine/Polypeptide zugeführt; das Zuführen der Proben kann beispielsweise mit einem Mikrodispenser zu jedem Messpunkt erfolgen. Bei diesem Ansatz ermöglicht der Reaktionsträger das Screenen zahlreicher verschiedener Proben auf einen bestimmten Bindungspartner für die auf dem Träger befindliche Sonde hin. So kann beispielsweise mit einer gegebenen Nukleinsäuresequenz (wie z. B. einem bestimmten Promotor) Probenmaterial daraufhin untersuchen, ob sie einen entsprechenden Bindungspartner für die fragliche Sequenz enthält.

Diese Art der Durchführung des Verfahrens lässt sich auch in Mikrotiterplatten durchführen, wobei jeder Vertiefung in einer Mikrotiterplatte eine verschiedene Probe zugeführt wird und so zahlreiche verschiedene Bindungsreaktionen parallel auf der Platte ablaufen können.

Bei einer weiteren bevorzugten Ausführungsform werden bei der Auswertung der erhaltenen Fluoreszenzsignale die Hintergrundsignale bei der Signalauswertung abgezogen. Die Hintergrundsignale sind im wesentlichen die Signale, die sich auf Grund der Fluoreszenz des Reaktionsträgers bzw. der darauf aufgebrachten Reagenzien, insbesondere Sonden, ergeben.

Es kann auch wünschenswert sein, die Sondenmoleküle selbst zu quantifizieren. In diesem Falle erhalten die Sondenmoleküle eine Markierung, die beispielsweise bei der Herstellung des Chips eingebaut werden kann, wenn das Sondenmolekül auf dem Träger synthetisiert wird. In diesem Fall hat man ein quantitatives Referenzsignal für das eigentliche Messsignal, welches auf der Fluoreszenz von gebundenen Probenmolekülen beruht. Eine solche Variante lässt sich beispielsweise derart durchführen, dass die markierte Sonde ein Fluoreszenzsignal in einem anderen Wellenlängenbereich erzeugt als das Probenmolekül. Durch gleichzeitiges Auslesen der beiden Signale, beispielsweise in UV für die Eigenfluoreszenz der Probe und im sichtbaren Bereich (z. B. für eine entsprechend markierte Sonde) lassen sich auch quantitative Bestimmungen von Probenmaterial besser durchführen.

Schließlich kann es bevorzugt sein, die Polarisation und Einstrahlwinkel des Anregungslichts zu variieren. Eine solche Maßnahme kann zusätzliche Information über das analysierte Protein auf dem Reaktionsträger liefern.

### Ablauf der Analyse:

Auf der Oberfläche eines Protein-Biochips ist eine Matrixförmige Anordnung verschiedener Messpunkte, enthaltend einen Bindungspartner für ein Protein, immobilisiert. An die immobilisierten Bindungspartner (Liganden) binden Moleküle aus der Probe. Mit dem Protein-Biochip können neben Protein-Protein-Wechselwirkungen auch andere Bindungsvorgänge untersucht werden, bei denen Proteine an andere Moleküle, wie beispielsweise Nukleinsäuren binden. Zum Beispiel ist für die Untersuchung der Genregulation die Bindung von Proteinen an Nukleinsäuren (speziell DNA) von Interesse. Mit dem erfindungsgemäßen Verfahren wird eine Probe daraufhin untersucht, ob sie ein bestimmtes Protein enthält. Der Chip kann demnach ein Array verschiedener Proteine (zur Untersuchung von Protein-Protein-Wechselwirkungen) oder ein DNA-Chip (zum Untersuchen von DNA-Protein-Wechselwirkungen) oder eine kombinatorische Bibliothek niedermolekularer Substanzen (z. B. zum Wirkstoffscreening) auf seiner Oberfläche tragen.

Die Immobilisierung der Liganden verläuft mit an sich bekannten Techniken, vgl. z. Bilitewski et al. in Methods in Biotechnology, Band 7: Affinity Biosensors, Seite 121 - 134. Jeder Messpunkt des Chips besteht aus einer Molekülspezies. Es können Chips mit wenigen Messpunkten bis zu einigen tausenden Punkten pro Quadratzentimeter erzeugt werden. Gängige Methoden zur Herstellung der Arrays sind u. a. die Microdosierung der Liganden in einer Lösung oder die On-Chip-Synthesen der Liganden mit photolithographischen Methoden.

Vor der Nachweisreaktion ist in der Regel eine Probenvorbehandlung (z. B. Extraktion, Zentrifugation, Aufreinigen der Polypeptide) erforderlich.

Nach dem Aufbringen der Probe auf den Chip findet die Anbindung derjenigen Probenmoleküle statt, für die sich spezifische Liganden auf dem Chip befinden. Die Erkennung dieses Bindungsvorgangs ist Voraussetzung für die abschließende Auswertung des Biochips. Die Messung wird in aller Regel nach der Anbindung und gegebenenfalls ein oder mehreren Wasch- und Spülschritten durchgeführt. Alternativ kann der Bindungsvorgang auch in Echtzeit verfolgt werden. Aus der Aussage über ein Bindungsereignis an einer Stelle des Chips folgt mit Erkenntnis der Chipbelegung die Information über das Vorhandensein eines bestimmten Polypeptids in der Probe.

Die Fluoreszenzanalyse bei einer vorgegebenen Wellenlänge im ultravioletten Spektralbereich erlaubt nun die Aussage über das Vorhandensein bestimmter UVaktiver Stoffe in den angebundenen Polypeptiden. Wie bereits ausgeführt, ist die Eigenfluoreszenz von Tryptophan bei der Anregung mit Licht von 280 nm Wellenlänge wegen der relativ hohen Quantenausbeute und dem relativ hohen Absorptionskoeffizienten besonders effizient. Da die meisten Proteine Tryptophan enthalten, ist dieser Nachweis praktisch auf beliebige Polypeptide anwendbar. Falls eine Quantifizierung der Signale gewünscht ist, ist es vorteilhaft, Kenntnisse über das zu bestimmende Polypeptid hinsichtlich dessen Tryptophangehalts zu besitzen. Die Signalstärke wird demnach nicht nur von der Konzentration des bindenden Proteins in der Probe, sondern auch von der Anzahl der Tryptophanreste in dem jeweiligen Protein bestimmt. Zu beachten ist auch die Beschaffenheit des Liganden. Falls dieser auch Tryptophanreste enthält, wird jeder Messpunkt ein Untergrundsignal zeigen, das von dem Messsignal abgezogen werden muss. Dieser Untergrund ist bei Kenntnis der immobilisierten Liganden bekannt. Insbesondere kann er vor der Zugabe der Probe durch eine Nullmessung bestimmt werden. Es ist jedoch vorteilhaft, dass die Liganden keine Eigenfluoreszenz im genannten Spektralbereich zeigen. Dies ist z. B. für DNA-Sonden gegeben.

Die Optiken sind in allen Fällen auf die relevanten Wellenlängenbereiche im UV abzustimmen. Insbesondere ist dabei die reduzierte Transparenz von gewünschten optischen Gläsern bei diesem Wellenlängenbereich zu berücksichtigen. Alle passiven optischen Bauelemente sind also z. B. mit speziellen Materialien wie z. B. Quarzglas auszuführen.

In Bezug auf die Lichtquellen erfordern die oben diskutierten verschiedenen Lösungen zum Ausführen des Biochips immer verschiedene Komponenten. Die Scannerversion erfordert einen Laser. In dem Wellenlängenbereich von ca. 280 nm sind folgende Laserquellen einsetzbar: Farbstofflaser, Excimerlaser oder frequenzverdoppelte Laserquellen. Dazu gehören z. B. vervierfachte Nd:YAG-Laser oder verdreifachte Halbleiterlaser. Im Falle der flächenhaften Ausleuchtung kommen auch Halogen- oder Gasentladungslampen in Frage, wobei die anregende Strahlung durch ein oder mehrere Interferenzbilder spektral auf die Absorptionswellen des zu untersuchenden Areals begrenzt wird. Dabei wird auch verhindert, dass Streulicht - trotz der Detektor-seitigen Filterung - einen Untergrund im detektierten Signal erzeugt. Die weiteren Verfahren sind sowohl mit Lasern als auch mit "breitbandingen" konventionellen Lichtquellen (wie einer thermischen Lichtquelle, z. B. Glühlampe oder Gasentladungslampe).

Zur Detektion können weitgehend Standardkomponenten verwendet werden. Die Empfindlichkeit z. B. einer CCD-Kamera nimmt zwar im UV gegenüber dem sichtbaren Spektralbereich etwas ab. Die Kameras sind dennoch für die vorliegenden Zwecke einsetzbar. Beim zeilenweisen Scannen können sowohl konventionelle Photodioden oder Pin-Dioden als auch Photomultiplier verwendet werden.

Die Nachweisgrenze des Systems wird wesentlich vom Hintergrund bestimmt, der einerseits durch die Eigenfluoreszenz einzelner Komponenten, insbesondere des Chipmaterials, erzeugt wird. Außerdem spielt das Streulicht im System eine Rolle. Im Vergleich zu Chipreadem, die im sichtbaren Spektralbereich arbeiten, ist die erhöhte UV-Aktivität dieser Materialien bei der vorliegenden Erfindung stärker zu berücksichtigen.

## Patentansprüche

1. Verfahren zum Bestimmen der Anwesenheit bzw. Abwesenheit mindestens eines Polypeptids/Proteins an mindestens einem vorgegebenen Messpunkt auf einem Reaktionsträger, **dadurch gekennzeichnet, dass** der Reaktionsträger einer elektromagnetischen Strahlung (Anregungslicht) ausgesetzt wird, die die Eigenfluoreszenz eines Polypeptids/Proteins anregen kann.

2. Verfahren nach Anspruch 1, **dadurch gekennzeichnet, dass** der Reaktionsträger ein Biochip oder ein Träger mit Vertiefungen in Art einer Mikrotiterplatte ist.

3. Verfahren nach einem der Ansprüche 1 oder 2, **dadurch gekennzeichnet, dass** das zu bestimmende Polypeptid/Protein keine Fremdmarkierung trägt.

4. Verfahren nach einem der Ansprüche 1 bis 3, **dadurch gekennzeichnet, dass** die Eigenfluoreszenz mindestens eines Tryptophan-, Tyrosin- und/oder Phenylalaninrestes in dem zu bestimmenden Polypeptid/Protein gemessen wird.

5. Verfahren nach einem der Ansprüche 1 bis 4, **dadurch gekennzeichnet, dass** das Anregungslicht eine Wellenlänge von c. 270 bis 290 nm, vorzugsweise ca.280 nm, aufweist.

6. Verfahren nach einem der Ansprüche 1 bis 5, **dadurch gekennzeichnet, dass** die Eigenfluoreszenz des Polypeptid/Protein mittels ortsaufgelöster Fluoreszenzmessung bestimmt wird.

7. Verfahren nach einem der Ansprüche 1 bis 6, **dadurch gekennzeichnet, dass** das Anregungslicht von einem Laser erzeugt wird.

8. Verfahren nach einem der Ansprüche 1 bis 7, **dadurch gekennzeichnet, dass** der Reaktionsträger einen planaren Lichtwellenleiter umfasst.

9. Verfahren nach einem der Ansprüche 1 bis 8, **dadurch gekennzeichnet, dass** das Anregungslicht auf eine der folgenden Arten dem Reaktionsträger zugeführt wird:
a) das Anregungslicht wird mittels einer Optik auf die gewünschte Position auf bzw. im Reaktionsträger gelenkt;
b) das Anregungslicht wird dem Reaktionsträger flächenhaft zugeführt;
c) das Anregungslicht wird in einen planaren Lichtwellenleiter eingekoppelt und gleichzeitig allen Messpunkten zugeführt.

10. Verfahren nach einem der Ansprüche 1 bis 9, **dadurch gekennzeichnet, dass** das durch das Anregungslicht erzeugte Fluoreszenzlicht wie folgt abgenommen wird:
a) das emitierte Fluoreszenzlicht wird mittels einer Optik einem Detektor zugeführt;
b) das emitierte Fluoreszenzlicht wird direkt einer Kamera zugeführt;
c) das emitierte Fluoreszenzlicht wird zeilenweise durch Scannen des Reaktionsträgers abgenommen und einem Detektor zugeführt;

11. Verfahren nach einem der Ansprüche 1 bis 10, **dadurch gekennzeichnet, dass** die Optik, mit der das Anregungslicht dem Reaktionsträger zugeführt wird, durchlässig ist für Licht einer Wellenlänge von ca. 270 bis 290 nm, vorzugsweise für Licht einer Wellenlänge von ca. 280 nm.

12. Verfahren nach einem der Ansprüche 1 bis 11, **dadurch gekennzeichnet, dass** das detektierte Fluoreszenzlicht eine Wellenlänge in dem Bereich von 300 bis 450 nm aufweist.

13. Verfahren nach einem der Ansprüche 1 bis 12, **dadurch gekennzeichnet, dass** entweder eine ortsauflösende Anregung oder eine ortsauflösende Detektion durchgeführt wird.

14. Verfahren nach einem der Ansprüche 1 bis 13, **dadurch gekennzeichnet, dass** sich in jedem Messpunkt gleichartige Sonden befinden und jedem Messpunkt eine verschiedene Probe zugeführt wird.

15. Verfahren nach einem der Ansprüche 1 bis 13, **dadurch gekennzeichnet, dass** jeder Messpunkt eine verschiedenartige Sonde enthält und jedem Messpunkt die gleiche Probe zugeführt wird.

16. Vorrichtung zum Durchführen eines Verfahrens nach einem der Ansprüche 1 bis 15, **dadurch gekennzeichnet, dass** die Optik, mit der das Anregungslicht dem Reaktionsträger zugeführt wird, durchlässig ist für ein Anregungslicht mit der Länge von 270 bis 290 nm, vorzugsweise für Anregungslicht mit der Wellenlänge von ca. 280 nm.

17. Vorrichtung nach Anspruch 16, **dadurch gekennzeichnet, dass** die Optik eine Quarzoptik umfasst.

18. Vorrichtung nach einem der Ansprüche 16 oder 17, **dadurch gekennzeichnet, dass** sie weiterhin einen Protein-Biochip enthält.

19. Reaktionsträger, vorzugsweise ein Biochip, geeignet zum Einsetzen in einem Verfahren nach einem der Ansprüche 1 - 15, **dadurch gekennzeichnet, dass** das Material, aus dem er im wesentlichen besteht, im wesentlichen keine Eigenfluoreszenz in dem Bereich von ca. 300 - 450 nm nach Bestrahlung mit einem Anregungslicht von ca. 270 - 290 nm zeigt.

20. Reaktionsträger nach Anspruch 19, **dadurch gekennzeichnet, dass** das Material ausgewählt wird aus Quarz, Keramik, Silizium und beschichtetem Glas, insbesondere Gold-beschichtetem Glas.

21. Reaktionsträger nach Anspruch 19 oder 20, **dadurch gekennzeichnet, dass** er mindestens ein Polypeptid in mindestens einem Messpunkt umfasst.
